# EUROPÄISCHE PATENTSCHRIFT

(11) **EP 2 934 619 B1**
(45) Veröffentlichungstag und Bekanntmachung des Hinweises auf die Patenterteilung: **08.03.2017**
(21) Anmeldenummer: 13818668.9
(22) Anmeldetag: 19.12.2013
(51) Int. Cl.: A61M 1/16, A61M 1/34, A61M 1/36

(54) **VORRICHTUNG ZUR ENTFERNUNG PROTEINGEBUNDENER TOXINE AUS BLUTPLASMA**
DEVICE FOR REMOVING PROTEIN-BOUND TOXINS FROM BLOOD PLASMA
DISPOSITIF D'ÉLIMINATION DES TOXINES LIÉES AUX PROTÉINES DANS LE PLASMA SANGUIN

(30) Priorität: 21.12.2012 DE 102012025164; 21.12.2012 US 201261740648 P
(43) Veröffentlichungstag der Anmeldung: 28.10.2015
(73) Patentinhaber: Fresenius Medical Care Deutschland GmbH, 61352 Bad Homburg (DE)
(72) Erfinder: TSCHULENA, Ulrich, 60439 Frankfurt (DE); JANKOWSKI, Joachim, 52159 Roetgen (DE); FABIG, Anselm, 15738 Zeuthen (DE)
(74) Vertreter: Behr, Wolfgang
(86) Internationale Anmeldenummer: PCT/EP2013/003867
(87) Internationale Veröffentlichungsnummer: WO 2014/095072

(56) Entgegenhaltungen:
- EP-A1- 2 087 916
- EP-A1- 2 446 908
- WO-A1-2013/004604
- RO-B1- 122 077
- US-A1- 2003 187 380
- US-A1- 2005 015 040
- US-A1- 2005 082 225

## Beschreibung

Die vorliegende Erfindung betrifft eine Vorrichtung zur Entfernung proteingebundener Toxine aus dem Blutplasma von Patienten unter Einwirkung eines elektromagnetischen Wechselfeldes und/oder eines elektrischen Gleichfeldes.

Die Aufgabe der gesunden Niere ist die Ausscheidung von Endprodukten des Stoffwechsels (harnpflichtige Substanzen) und Giftstoffen (Urämietoxine) aus dem Körper durch Bildung des Harns. Die Niere entfernt dabei ein breites Spektrum an Substanzen unterschiedlichen Molekulargewichts. Eine Übersicht über urämische Toxine wurde von R. Vanholder *et al*. veröffentlicht. (R. Vanholder et al., Kidney International, 63 (2003) 1934 - 1943) Die urämischen Toxine werden an Hand ihres Molekulargewichts in drei Klassen eingeteilt. Toxine mit einem Molekulargewicht von unter 500 Dalton bilden die Gruppe mit niedrigem Molekulargewicht. Die Mittelmoleküle liegen in einem mittleren Bereich mit einem Molekulargewicht zwischen 500 und 12 000 D. Zu den Mittelmolekülen gehört beispielsweise β₂-Microglobulin (11800 D). Die dritte Klasse von Urämietoxinen bilden Moleküle mit einem Molekulargewicht von über 12 000 D.

Darüber hinaus wird nach der Wasserlöslichkeit der Urämietoxine unterschieden. Beispiele für gut wasserlösliche urämische Toxine mit einem niedrigen Molekulargewicht sind Harnstoff, Creatinin, Oxalate, Guanidine und Harnsäure.

Beispiele für schlecht wasserlösliche urämische Toxine sind *p*-Cresol, Indoxylsulfat, Phenol, Hippursäure und Homocystein. Diese Urämietoxine liegen im Serum überwiegend an Proteinen gebunden vor.

Bei gesunden Personen werden die Urämietoxine über die Niere mit dem Harn ausgeschieden. Beim chronischen Nierenversagen verbleiben die urämischen Toxine jedoch im Blut des Patienten und müssen durch Hämodialyse oder Peritonealdialyse entfernt werden.

Während die Entfernung wasserlöslicher Toxine wie beispielsweise Harnstoff oder Creatinin mit den bekannten Dialyseverfahren, wie zum Beispiel Hämodialyse, sehr gut möglich ist, ist die Entfernung von schlecht wasserlöslichen hydrophoben Urämietoxinen mittels Hämodialyseverfahren durch die Proteinbindung stark erschwert. Man geht allgemein davon aus, dass ein chemisches Gleichgewicht zwischen dem freien, gelösten Toxin und dem proteingebundenen Toxin vorliegt, das weit auf Seiten des proteingebundenen Toxins liegt. Dies bedeutet, dass der überwiegende Teil dieser Urämietoxine an Proteine gebunden ist und nur ein kleiner Teil im Blutplasma gelöst ist.

Da es sich bei einem großen Teil der Substanzen um niedermolekulare Komponenten handelt, die zu einem geringen Teil in freier Form vorliegen, sind sie prinzipiell dialysierbar.

Weiterhin nimmt man an, dass Plasmaproteine, insbesondere Albumin, als Bindungspartner der hydrophoben Urämietoxine fungiert. Albumin wird auf Grund seines Molekulargewichtes von Dialysemembranen zurückgehalten. Albumin wird durch Hämodialyseverfahren also nicht entfernt. Damit kann nur der freie, gelöste Anteil der urämischen Toxine aus dem Blut des Patienten entfernt werden. Die Einstellung des Gleichgewichtes unter der Dialyse wird zum geschwindigkeitsbestimmenden Schritt. Es ist zwar zu erwarten, dass sich nach der Entfernung der gelösten Toxine aus dem Blut das Gleichgewicht zwischen freien und proteingebundenen Toxinen wieder neu einstellt und bei genügend langer Dialysezeit ein beträchtlicher Teil der Toxine entfernen lässt, diese Zeit steht bei Hämodialysebehandlungen jedoch nicht zur Verfügung.

Es besteht also ein Bedarf an Dialyseverfahren, die auch die proteingebundenen urämischen Toxine aus dem Blut des Patienten entfernen.

Die vorliegende Erfindung liegt somit die Aufgabe zu Grunde, eine neue Vorrichtung zur Entfernung von proteingebundenen Toxinen aus Blutplasma bereitzustellen. Ferner ist es eine Aufgabe der Erfindung, ein entsprechendes Verfahren anzugeben. Die vorliegende Erfindung betrifft eine Vorrichtung gemäß dem Oberbegriff von Anspruch 1 zur Entfernung proteingebundener Toxine aus dem Blutplasma von Patienten unter Einwirkung eines hochfrequenten elektrischen oder elektromagnetischen Wechselfeldes und/oder eines elektrischen Gleichfeldes.

Folgende Dokumente offenbaren Vorrichtungen gemäß dem Stand der Technik: US2005/0015040 A1, US2005/008225 A1 und WO2013/004604 A1. Erfindungsgemäß umfasst die Vorrichtung einen ersten extrakorporalen Kreislauf zur Aufnahme von zu reinigendem Blut und einen zweiten Kreislauf mit einem Hämodialysator und/oder Hämofilter. Beide Kreisläufe sind über einen Plasmafilter verbunden, der eine ungefilterte und eine gefilterte Seite aufweist. Die ungefilterte Seite wird von der gefilterten Seite durch zumindest ein Filtermaterial, beispielsweise eine Filtermembran, getrennt, wobei ein Flüssigkeitszuführeingang der ungefilterten Seite mit einer dritten mit dem Patienten verbindbaren Leitungseinrichtung verbunden ist, ein Flüssigkeitsabführausgang der ungefilterten Seite mit einer vierten mit dem Patienten verbindbaren Leitungseinrichtung verbunden ist, ein Flüssigkeitsabführausgang der gefilterten Seite mit der ersten Leitungseinrichtung verbunden ist und ein Flüssigkeitszuführeingang der gefilterten Seite mit der zweiten Leitungseinrichtung verbunden ist. Somit ist die erste Leitungseinrichtung über die dritte Leitungseinrichtung und die zweite Leitungseinrichtung über die vierte Leitungseinrichtung mit dem Patienten verbindbar. Der Plasmafilter weist vorzugsweise einen Druckgradienten auf, so dass im vorderen Bereich des Filters filtriert wird (Flüssigkeitsübertritt von der ungefilterten zur gefilterten Seite) und im hinteren Bereich des Filters rückfiltiert wird (Flüssigkeitsübertritt von der gefilterten auf die ungefilterte Seite), d.h. das gereinigte Blutplasma wird dem Patienten wieder zugeführt. Alternativ zum Plasmafilter kann in einer nicht beanspruchten Vorrichtung ein Zellseparator zur Trennung von Blut in Plasma und zelluläre Bestandteile eingebaut sein. Dieser Zellseparator kann beispielsweise eine Zentrifuge sein.

Der Filter kann die zellulären Bestandteile des Blutes zurückhalten, wobei Albumin sowie andere Plasmaproteine mit dem daran gebundenen Urämietoxinen sowie weitere kleinmolekulare Substanzen mit dem Blutplasma durchgelassen werden. Somit kann der Plasmaprotein-Toxin-Komplex in die erste Leitungseinrichtung gelangen, die Urämietoxine entfernt werden und das gereinigte Blutplasma anschließend über die zweite bzw. vierte Leitungseinrichtung dem Patienten zugeführt werden.

Die Einwirkung eines elektromagnetischen Wechselfeldes und/oder eines elektrischen Gleichfeldes kann auf der Seite des plasmahaltigen Filtrats als auch auf der Seite der angereicherten Flüssigkeit erfolgen. Eine Bestrahlung auf der Seite des plasmahaltigen Filtrats bietet den Vorteil, dass das elektromagnetische Wechselfeld und/oder das elektrische Gleichfeld nicht auf die Blutzellen einwirkt. Damit wird die Biokompatiblität des Verfahrens erhöht.

Die Reinigung des Blutplasmas erfolgt durch einen Adsorber und/oder einen Dialysator oder Hämofilter.

Ferner weist die Vorrichtung ein Mittel zum Erzeugen eines hochfrequenten elektrischen oder elektromagnetischen Wechselfeldes und/oder eine Einrichtung zur Erzeugung einen elektrischen Gleichfeldes, wobei das zu reinigende Blut vor und/oder während des Kontaktes mit dem Adsorber oder dem Dialysator/Hämofilter dem hochfrequenten, elektromagnetischen Wechselfeld und/oder dem elektrischen Gleichfeld ausgesetzt wird. Die vorliegende Erfindung stellt damit ein Verfahren bereit, dass die Lage des Gleichgewichts zwischen freien und proteingebundenen Toxinen verschiebt und die Einstellung des Gleichgewichts während der Dialysebehandlung beschleunigt.

Der Erfindung liegt die Erkenntnis zugrunde, dass die Bindungen zwischen Urämietoxinen und Plasmaproteinen in der Regel keine "echten" chemischen (kovalenten) Bindungen sind, sondern es sich um reversible Bindungen handelt. Diese Bindungen beruhen im Wesentlichen auf den elektrostatischen Eigenschaften und Wechselwirkungen der beteiligten Moleküle. Es wurde gefunden, dass die Stärke dieser Bindungen oder Wechselwirkungen erfindungsgemäß durch die Einwirkung von hochfrequenten elektrischen oder elektromagnetischen Feldern herabgesetzt werden kann. Durch die Verwendung hochfrequenter elektrischer oder elektromagnetischer Felder und/oder elektrischer Gleichfelder während der Dialyse kann der Anteil proteingebundener Urämie-Toxine deutlich reduziert werden. Im Rahmen der im klinischen Alltag durchgeführten Dialyse kann durch zusätzliche Anwendung hochfrequenter elektrischer oder elektromagnetischer Felder und/oder elektrischer Gleichfelder die Freisetzungsrate proteingebundener Urämietoxine aus der Proteinbindung erhöht werden. Dadurch wird während der Dialyse eine verbesserte Abtrennung dieser Substanzen aus dem Blut des Patienten erreicht. Die betreffenden Urämietoxine können somit vermehrt und effektiver dialysiert werden.

Verfahren zur Hämodialyse und zur Hämofiltration sind dem Fachmann bekannt. In den Veröffentlichungen "Replacement of Renal Function by Dialysis" (Drukker, Parsons und Mäher; Kluwer Academic Publishers, 4. Auflage 1996; und "Hemodialysis Machines and Monitors" von H.-D. Polaschegg und N.W. Levin) - auf deren Offenbarung hiermit explizit verwiesen wird - findet sich eine Zusammenfassung der wichtigsten Hämodialyseverfahren und -maschinen: Bei der Hämodialyse wird das Blut eines Patienten durch eine arterielle Blutleitung in die Blutkammer eines Dialysators geleitet. Das Blut wird normalerweise mit Hilfe einer in der arteriellen Blutleitung angeordneten peristaltischen Rotationspumpe transportiert. Nach Durchgang durch die Pumpe wird das Blut durch die Blutkammer des Dialysators geleitet und schließlich durch eine venöse Tropfkammer und eine damit verbundene venöse Blutleitung zu dem Patienten zurückgeleitet. Ein Venendruckmonitor ist mit der venösen Tropfkammer als Schutzsystem zur unmittelbaren Erfassung eines Blutverlustes an die Umgebung verbunden. Gegebenenfalls können bei der sogenannten Einnadeldialyse zwei für die arterielle und die venöse Kanüle erforderliche Nadeln durch eine einzige Nadel ersetzt werden. Bei dieser Art der Dialyse besteht der extrakorporale Kreislauf aus einer Einnadelkanüle mit angeschlossenem Y-Stück. Von dem Dialysator führt die venöse Leitung zurück zu dem Y-Stück. Die arterielle und die venöse Leitung werden abwechselnd durch Klemmen verschlossen. Es laufen eine oder mehrere Blutpumpen, um für die abwechselnde Strömung zu und von dem Y-Stück zu sorgen.

Bei der Hämodialyse erfolgt die Entfernung der gelösten Stoffe aus dem Blut durch Diffusion durch die Dialysatormembran. Wenngleich zur Ultrafiltration des überschüssigen Wassers eines Patienten zusätzlich ein geringer transmembranöser Druck aufgebracht wird, spielt diese Filtration kaum eine Rolle für die Reinigung des Blutes von speziellen Substanzen.

Die Entfernung gelöster Stoffe bei der Hämofiltration erfolgt durch Konvektion und nicht durch Diffusion. Gleichzeitig wird das Ultrafiltrat fast vollständig durch eine Substitutionsflüssigkeit ersetzt, die eine ähnliche Zusammensetzung hat wie das Dialysat bei der Dialyse. Bei diesem Verfahren wird die Ähnlichkeit mit der natürlichen Niere und die wirksamere Entfernung größerer Moleküle betont. Die Entfernung niedermolekularer Substanzen ist dagegen gegenüber der Hämodialyse verringert, weil höchstens 45% des Blutes bei der sogenannten Nachverdünnungshämofiltration ultrafiltriert werden können.

Hämodiafiltration, eine Kombination aus Hämodialyse und Hämofiltration, kann durchgeführt werden, indem die extrakorporalen Kreisläufe einer Hämofiltrations- und einer Hämodialysemaschine kombiniert werden. Hämodialysemaschinen mit volumetrisch gesteuerter Ultrafiltration können leicht zur Hämodiafiltration angepasst werden, die kostengünstiger ist. Diese ist besonders kostengünstig, wenn die Substitutionsflüssigkeit online aus der Dialyseflüssigkeit hergestellt wird.

Zusammenfassend lässt sich sagen, dass bei der Hämodialyse das Blut des Patienten gereinigt wird, indem die zu entfernenden Substanzen des Blutes aufgrund eines Konzentrationsgefälles über die Membran des Dialysators durch die Membran diffundieren und dadurch die Dialyseflüssigkeit erreichen. Die treibende Kraft bei der Hämofiltration ist im Wesentlichen ein Druckunterschied über die Membran, der einen konvektiven Transport von Substanzen durch die Membran bewirkt und dabei das Blut vor allem auch von höhermolekularen Substanzen reinigt. Bei der Hämofiltration sowie bei dem kombinierten Verfahren der Hämodiafiltration wird Flüssigkeit aus dem Blut des Patienten entfernt, die bis auf einen kleinen Differenzbetrag zur Steuerung des Flüssigkeitsausgleichs substituiert werden muss. Die erfindungsgemäße Vorrichtung kann ebenfalls in oder nach dem Mittel zum Erzeugen eines hochfrequenten, elektromagnetischen Wechselfeldes und/oder eine Einrichtung zur Erzeugung einen elektrischen Gleichfeldes einen Adsorber aufweisen. Dieser Adsorber kann beispielsweise ein Anionenaustauscher oder ein unspezifischer Adsorber sein. Während der Anionenaustauscher Bilirubin, Gallensäuren und andere Ionen bindet, kann der unspezifische Adsorber, beispielsweise ein Neutralharz oder Aktivkohle, andere proteingebundene Toxine zurückhalten.

Die erfindungsgemäße Vorrichtung gemäß dem Oberbegriff von Anspruch 1 weist zusätzlich Mittel zur Erzeugung eines Feldes auf. Das Feld kann ein hochfrequentes elektrisches Feld, ein hochfrequentes elektromagnetisches Feld und/oder ein elektrisches Gleichfeldes sein. Dem Fachmann sind Mittel zur Erzeugung solcher Felder bekannt.

Die erfindungsgemäße Vorrichtung kann zur Erzeugung eines hochfrequenten, elektrischen oder elektromagnetischen Feldes beispielsweise einen Hochfrequenzkondensator, eine Hochfrequenzspule und/oder eine Hochfrequenzelektrode aufweisen. Das hochfrequente elektrische Feld oder das hochfrequente elektromagnetische Feld weist eine Frequenz von 100 kHz bis 2 GHz, vorzugsweise 1 MHz bis 1 GHz, auf. Es kann beispielsweise ein hochfrequentes elektromagnetisches Feld verwendet werden, welches eine magnetische Flussdichte aufweist von kleiner oder gleich 100 mTesla, bevorzugt von 0,001 bis 100 mTesla, besonders bevorzugt von 0,01 bis 10 mTesla, insbesondere von 0,01 bis 2 mTesla. Zur Lösung der Bindung zwischen Plasmaprotein und Urämie-Toxin wird erfindungsgemäß ein hochfrequentes elektrisches oder elektromagnetisches Feld verwendet. Dabei kann das hochfrequente elektromagnetische Feld eine Frequenz aufweisen von 100 kHz bis 1 GHz, bevorzugt von 0,5 MHz bis 100 MHz, besonders bevorzugt von 1 MHz bis 50 MHz, ganz besonders bevorzugt von 1 MHz bis 20 MHz. Das zu reinigende Blut kann dabei einem hochfrequenten elektrischen oder elektromagnetischen Feld ausgesetzt werden, welches im Wesentlichen über die Zeit eine konstante Frequenz aufweist. Alternativ kann das hochfrequente elektrische oder elektromagnetische Feld eine variierende Frequenz aufweisen, wobei die Frequenz und/oder Feldstärke in einem regelmäßigen oder einem unregelmäßigen Muster variiert werden kann. In einer beispielhaften Ausführungsform wird das zu reinigende Blut einem hochfrequenten elektromagnetischen Feld ausgesetzt welches mit einer relativ geringen Frequenz beginnt und dessen Frequenz über die Zeit bis zu einer vorher festgelegten maximalen Frequenz gesteigert wird. Alternativ dazu kann das zu reinigende Blut auch einem hochfrequenten elektrischen oder elektromagnetischen Feld ausgesetzt werden, welches mit einer hohen Maximalfrequenz beginnt und dessen Frequenz über die Zeit bis zu einer vorher festgelegten Mindestfrequenz verringert wird. Durch die Verwendung eines hochfrequenten elektromagnetischen Feldes mit variierenden Frequenzen wird die Effektivität der Lösung von Bindungen zwischen Urämie-Toxin und Plasmaprotein verbessert.

Weiterhin kann die erfindungsgemäße Vorrichtung Mittel zur Erzeugung eines elektrischen Gleichfeldes aufweisen. Dem Fachmann sind solche Vorrichtungen bekannt. Die erfindungsgemäße Vorrichtung kann beispielsweise aus einem Plattenkondensator mit zwei, vier oder mehr Platten aufgebaut sein. Das elektrische Gleichfeld weist eine Feldstärke von 10 V/m bis 400 V/m, besonders bevorzugt 100 V/m bis 250 V/m, auf.

Die Mittel zur Erzeugung eines hochfrequenten elektrischen oder elektromagnetischen Feldes und/oder eines elektrischen Gleichfeldes können derart ausgestaltet sein und im bzw. am Blutplasmakreislauf angeordnet sein, dass das zu reinigende Blutplasma dem Feld ausgesetzt werden kann vor, während oder sowohl vor als auch während des Kontaktes des zu reinigenden Blutplasmas mit dem Dialysator (18) bzw. mit dem Adsorber (19).

In einer bevorzugten Ausführungsform der Vorrichtung nach Anspruch 1 werden Mittel zur Erzeugung eines hochfrequenten elektrischen oder elektromagnetischen Feldes verwendet.

In einer weiteren bevorzugten Ausführungsform der Vorrichtung nach Anspruch 1 werden Mittel zur Erzeugung eines hochfrequenten elektrischen oder elektromagnetischen Feldes und Mittel zur Erzeugung eines elektrischen Gleichfeldes verwendet.

Eine mit dem Patienten verbindbare Leitungseinrichtungen (24 und 27) können direkt mit dem Patienten verbunden sein. Alternativ können optional weitere Leitungseinrichtungen vorgesehen sein über welche der Patient den mit Leitungseinrichtungen (24 und 27) in Flüssigkeitsverbindung steht.

Die Körperflüssigkeitsfördereinrichtung (16) ist hinsichtlich ihrer Flüssigkeitsförderrate steuerbar und kann somit einen regulierbaren Durchfluss der Körperflüssigkeit bzw. Flüssigkeit durch die Vorrichtung erzeugen. Die Flüssigkeitsförderrate liegt in einem Bereich von 100 bis 500 ml/min. In einer bevorzugten Ausführungsform liegt sie bei etwa 300 ml/min. Die Körperflüssigkeitsfördereinrichtung kann eine Pumpe sein, beispielsweise eine Membranpumpe oder eine Peristaltikpumpe.

Eine Eliminierung von proteingebundenen Toxinen findet insbesondere auch durch die Leber statt. Daher ist es denkbar, die erfindungsgemäße Vorrichtung auch bei der Behandlung des akuten oder chronischen Leberversagens zu verwenden.

Weitere Einzelheiten und Vorteile der Erfindung ergeben sich anhand der nachfolgend erläuterten Figuren und Ausführungsbeispiele. In den Figuren zeigen:
- Figur 1:: ein schematisches Blockschaltbild einer Ausführungsform einer erfindungsgemäßen Vorrichtung;
- Figur 2:: ein schematisches Blockschaltbild einer weiteren Ausführungsform der erfindungsgemäßen Vorrichtung;
- Figur 3:: ein schematisches Blockschaltbild einer weiteren Ausführungsform der erfindungsgemäßen Vorrichtung;
- Figur 4:: ein schematisches Blockschaltbild einer weiteren Ausführungsform der erfindungsgemäßen Vorrichtung;
- Figur 5:: ein schematisches Blockschaltbild einer weiteren Ausführungsform der erfindungsgemäßen Vorrichtung;
- Figur 6:: ein schematisches Blockschaltbild einer weiteren Ausführungsform der erfindungsgemäßen Vorrichtung;
- Figur 7:: experimentelle Ergebnisse betreffend den Einfluss hochfrequenter elektromagnetischer Felder auf den proteingebundenen Anteil der Urämietoxine;
- Figur 8:: experimentelle Ergebnisse als Nachweis der fehlenden Beschädigung der Membran durch die hochfrequenten Felder;
- Figur 9:: experimentelle Ergebnisse betreffend die Einflüsse eines HF-Feldes im Frequenzbereich 1 bis 170 MHz auf den proteingebundenen Anteil der Urämietoxine;
- Figur 10:: experimentelle Ergebnisse betreffend die Einflüsse eines HF-Feldes im Frequenz-Bereich 110 bis 115 MHz auf den proteingebundenen Anteil der Urämietoxine;
- Figur 11:: experimentelle Ergebnisse betreffend die Einflüsse eines H-Feldes in den Frequenzbereichen 1 bis 6 MHz sowie 9 bis 13 MHz auf den proteingebundenen Anteil der Urämietoxine; und
- Figur 12:: experimentelle Ergebnisse betreffend die Einflüsse der Feldstärke auf den proteingebundenen Anteil der Urämietoxine.

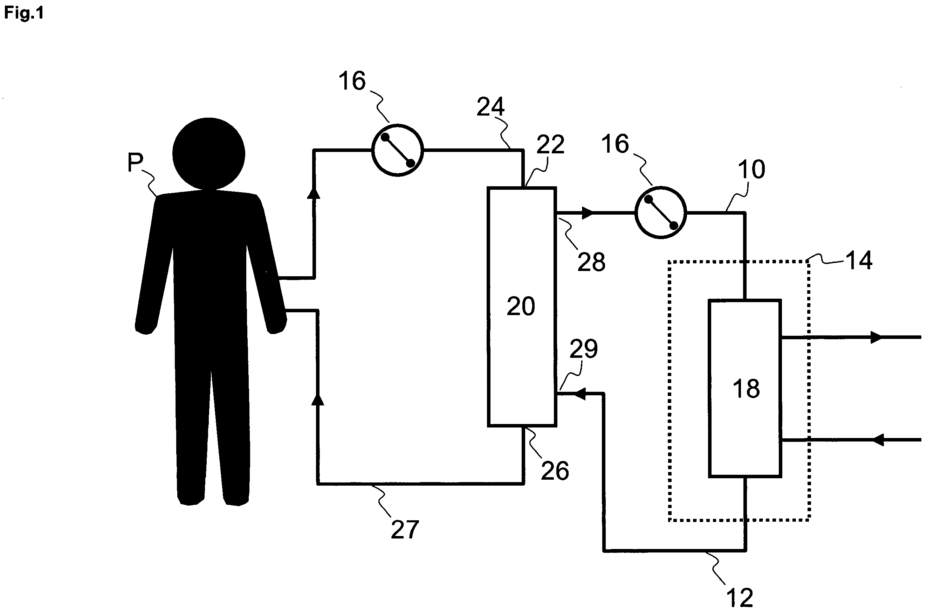

In Figur 1 ist eine Ausführungsform der erfindungsgemäßen Vorrichtung schematisch dargestellt. Die Bezugszeichen dieser Figur sind wie nachfolgend wiedergegeben zugeordnet:
- 10: erste Leitungseinrichtung
- 12: zweite Leitungseinrichtung
- 14: Mittel zum Erzeugen eines Feldes
- 16: Flüssigkeitsfördereinrichtung
- 18: Hämodialysator oder Hämofilter mit Zu- und Ableitungen für eine Dialysierflüssigkeit
- 20: Filter/Zellseparator
- 24: dritte Leitungseinrichtung
- 27: vierte Leitungseinrichtung
- 22: Flüssigkeitszuführeingang der ungefilterten Seite
- 26: Flüssigkeitsabführausgang der ungefilterten Seite
- 28: Flüssigkeitsabführausgang der gefilterten Seite
- 29: Flüssigkeitszuführeingang der gefilterten Seite
- P: Patient

Die dritte Leitungseinrichtung 24 weist ein erstes und ein zweites Ende auf, wobei das erste Ende mit dem Patienten P verbindbar ist und das zweite Ende mit dem Flüssigkeitszuführeingang 22 einer ungefilterten Seite eines Filters 20 verbunden ist. Der Plasmafilter 20 weist neben der ungefilterten Seite eine gefilterte Seite auf, wobei die ungefilterte Seite von der gefilterten Seite durch zumindest ein Filtermaterial getrennt ist. Die vierte Leitungseinrichtung 27 weist ein erstes und ein zweites Ende auf, wobei das erste Ende mit dem Flüssigkeitsabführausgang 26 einer ungefilterten Seite eines Filters 20 verbunden ist und das zweite Ende mit dem Patienten P verbindbar ist. Das Vollblut, welches durch den Flüssigkeitszuführeingang 22 der ungefilterten Seite eintritt, kann als Blutplasma teilweise durch den Flüssigkeitsabführausgang 28 der gefilterten Seite wieder austreten. Über die erste Leitungseinrichtung 10 wird dann das abgetrennte Blutplasma durch einen Dialysator 18 geleitet, der von einem Mittel zum Erzeugen eines Feldes 14 zumindest teilweise umgeben ist. Unter Einwirkung des Feldes werden die proteingebundenen Toxine zumindest teilweise freigesetzt und über die Membran des Dialysators 18 abgetrennt. Anschließend fließt das bestrahlte und gereinigte Blutplasma über die zweite Leitungseinrichtung 12 und tritt am Flüssigkeitszuführeingang 29 der gefilterten Seite wieder in den Plasmafilter 20 ein und durch den Flüssigkeitsabführausgang 26 der ungefilterten Seite in die vierte Leitungseinrichtung aus. In der vierten Leitungseinrichtung 27 treffen somit das ungefilterte Vollblut und das bestrahlte und gereinigte Blutplasma wieder aufeinander und werden in den Patienten P zurückgeleitet.

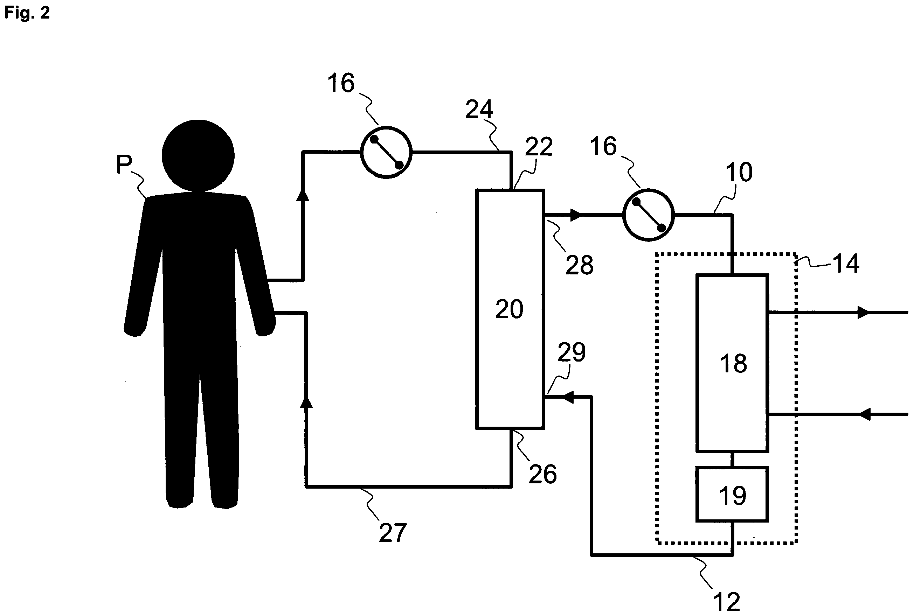

In Figur 2 ist eine weitere Ausführungsform der erfindungsgemäßen Vorrichtung schematisch dargestellt. Zur obenstehend beschriebenen Ausführungsform identische Teile sind mit identischen Bezugszeichen bezeichnet. Zusätzlich befindet sich in dieser Ausführungsform der erfindungsgemäßen Vorrichtung stromabwärts vom Dialysator 18 innerhalb des Feldes 14 ein Adsorber 19.

Die dritte Leitungseinrichtung 24 gemäß der in Figur 2 dargestellten Ausführungsform weist ein erstes und ein zweites Ende auf, wobei das erste Ende mit dem Patienten P verbindbar ist und das zweite Ende mit dem Flüssigkeitszuführeingang 22 einer ungefilterten Seite eines Filters 20 verbunden ist. Der Plasmafilter 20 weist neben der ungefilterten Seite eine gefilterte Seite auf, wobei die ungefilterte Seite von der gefilterten Seite durch zumindest ein Filtermaterial getrennt ist. Die vierte Leitungseinrichtung 27 weist ein erstes und ein zweites Ende auf, wobei das erste Ende mit dem Flüssigkeitsabführausgang 26 einer ungefilterten Seite eines Filters 20 verbunden ist und das zweite Ende mit dem Patienten P verbindbar ist. Das Vollblut, welches durch den Flüssigkeitszuführeingang 22 der ungefilterten Seite eintritt, kann als Blutplasma teilweise durch den Flüssigkeitsabführausgang 28 der gefilterten Seite wieder austreten. Über die erste Leitungseinrichtung 10 wird dann das abgetrennte Blutplasma durch einen Dialysator 18 und einen Adsorber 19 geleitet, die beide von einem Mittel zum Erzeugen eines Feldes 14 zumindest teilweise umgeben ist. Unter Einwirkung des Feldes werden die proteingebundenen Toxine zumindest teilweise freigesetzt und über die Membran des Dialysators 18 abgetrennt. Anschließend fließt das Blutplasma weiter den Adsorber 19, der die im Feld freigesetzten Toxine aufnimmt. Anschließend fließt das Blutplasma in die zweite Leitungseinrichtung 12 und tritt am Flüssigkeitszuführeingang 29 der gefilterten Seite wieder in den Plasmafilter 20 ein und durch den Flüssigkeitsabführausgang 26 der ungefilterten Seite in die vierte Leitungseinrichtung aus. In der vierten Leitungseinrichtung 27 treffen somit das ungefilterte Vollblut und das bestrahlte und gereinigte Blutplasma wieder aufeinander. Diese Vorrichtung hat den Vorteil, dass neben dem Dialysator 18 zusätzlich ein Adsorber 19 die aus den Plasmaproteinen freigesetzten Toxine bindet. Die Anordnung des Adsorbers 19 im Plasmakreislauf hat den weiteren Vorteil, dass aus dem Adsorber freigesetztes Adsorbermaterial vom Plasmafilter zurückgehalten wird und nicht in den Vollblutkreislauf (24, 27) gelangen kann.

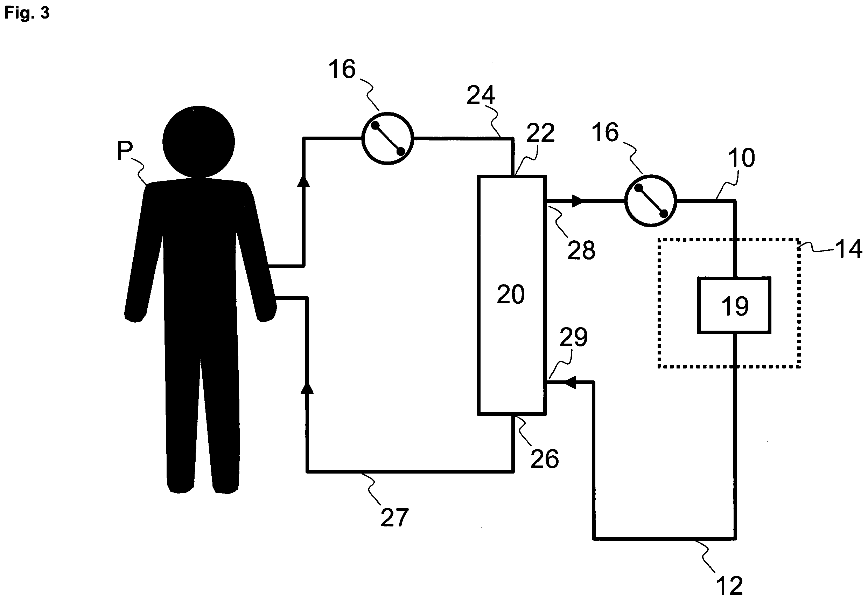

In Figur 3 ist eine Ausführungsform der erfindungsgemäßen Vorrichtung schematisch dargestellt. Zu den obenstehend beschriebenen Ausführungsformen identische Teile sind mit identischen Bezugszeichen bezeichnet. An Stelle des Dialysators 18 befindet sich in dieser Ausführungsform der erfindungsgemäßen Vorrichtung innerhalb des Feldes 14 ein Adsorber 19.

Die dritte Leitungseinrichtung 24 gemäß der in Figur 3 dargestellten Ausführungsform weist ein erstes und ein zweites Ende auf, wobei das erste Ende mit dem Patienten P verbindbar ist und das zweite Ende mit dem Flüssigkeitszuführeingang 22 einer ungefilterten Seite eines Filters 20 verbunden ist. Der Plasmafilter 20 weist neben der ungefilterten Seite eine gefilterte Seite auf, wobei die ungefilterte Seite von der gefilterten Seite durch zumindest ein Filtermaterial getrennt ist. Die vierte Leitungseinrichtung 27 weist ein erstes und ein zweites Ende auf, wobei das erste Ende mit dem Flüssigkeitsabführausgang 26 einer ungefilterten Seite eines Filters 20 verbunden ist und das zweite Ende mit dem Patienten P verbindbar ist. Das Vollblut, welches durch den Flüssigkeitszuführeingang 22 der ungefilterten Seite eintritt, kann als Blutplasma teilweise durch den Flüssigkeitsabführausgang 28 der gefilterten Seite wieder austreten. Über die erste Leitungseinrichtung 10 wird dann das abgetrennte Blutplasma durch einen Adsorber 19 geleitet, der von einem Mittel zum Erzeugen eines Feldes 14 zumindest teilweise umgeben ist. Unter Einwirkung des Feldes werden die proteingebundenen Toxine zumindest teilweise freigesetzt und vom Adsorber 19 aufgenommen. Anschließend fließt das bestrahlte und gereinigte Blutplasma über die zweite Leitungseinrichtung 12 und tritt am Flüssigkeitszuführeingang 29 der gefilterten Seite wieder in den Plasmafilter 20 ein und durch den Flüssigkeitsabführausgang 26 der ungefilterten Seite in die vierte Leitungseinrichtung 27 aus. In der vierten Leitungseinrichtung 27 treffen somit das ungefilterte Vollblut und das bestrahlte und gereinigte Blutplasma wieder aufeinander.

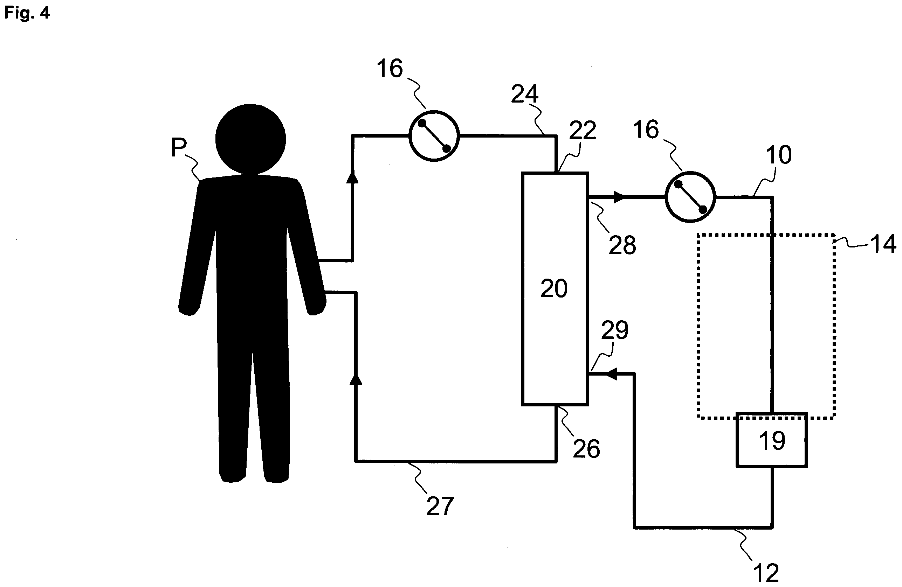

In Figur 4 ist eine Ausführungsform der erfindungsgemäßen Vorrichtung schematisch dargestellt. Zu den obenstehend beschriebenen Ausführungsformen identische Teile sind mit identischen Bezugszeichen bezeichnet. In dieser Ausführungsform der erfindungsgemäßen Vorrichtung befindet sich der Adsorber 19 stromabwärts teilweise außerhalb des Feldes 14.

Die dritte Leitungseinrichtung 24 gemäß der in Figur 4 dargestellten Ausführungsform weist ein erstes und ein zweites Ende auf, wobei das erste Ende mit dem Patienten P verbindbar ist und das zweite Ende mit dem Flüssigkeitszuführeingang 22 einer ungefilterten Seite eines Filters 20 verbunden ist. Der Plasmafilter 20 weist neben der ungefilterten Seite eine gefilterte Seite auf, wobei die ungefilterte Seite von der gefilterten Seite durch zumindest ein Filtermaterial getrennt ist. Die vierte Leitungseinrichtung 27 weist ein erstes und ein zweites Ende auf, wobei das erste Ende mit dem Flüssigkeitsabführausgang 26 einer ungefilterten Seite eines Filters 20 verbunden ist und das zweite Ende mit dem Patienten P verbindbar ist. Das Vollblut, welches durch den Flüssigkeitszuführeingang 22 der ungefilterten Seite eintritt, kann als Blutplasma teilweise durch den Flüssigkeitsabführausgang 28 der gefilterten Seite wieder austreten. Über die erste Leitungseinrichtung 10 wird dann das abgetrennte Blutplasma durch einen Adsorber 19 geleitet, der von einem Mittel zum Erzeugen Feldes 14 nur teilweise umgeben ist. Unter Einwirkung des Feldes werden die proteingebundenen Toxine zumindest teilweise freigesetzt und vom Adsorber 19 aufgenommen. Anschließend fließt das bestrahlte und gereinigte Blutplasma über die zweite Leitungseinrichtung 12 und tritt am Flüssigkeitszuführeingang 29 der gefilterten Seite wieder in den Plasmafilter 20 ein und durch den Flüssigkeitsabführausgang 26 der ungefilterten Seite in die vierte Leitungseinrichtung 27 aus. In der vierten Leitungseinrichtung 27 treffen somit das ungefilterte Vollblut und das bestrahlte und gereinigte Blutplasma wieder aufeinander.

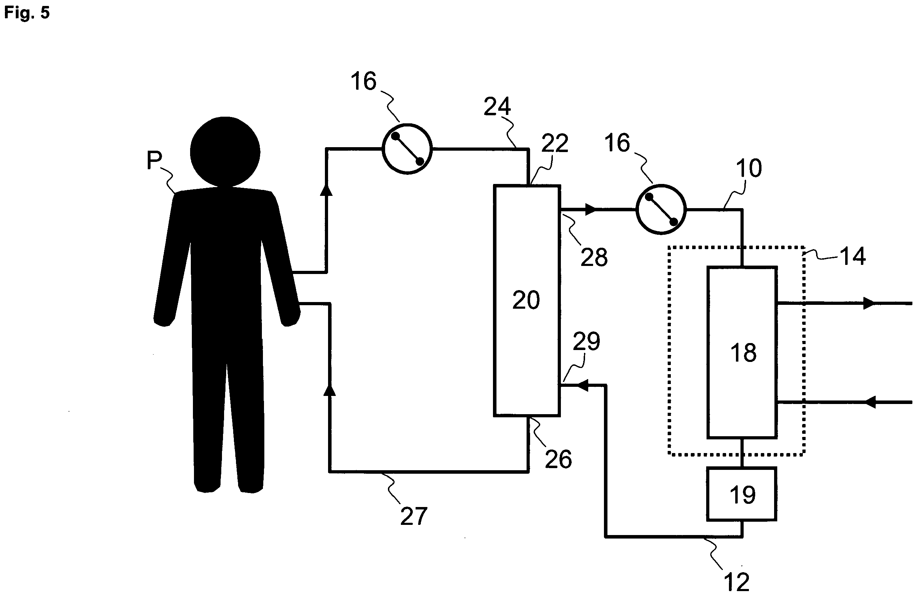

In Figur 5 ist eine weitere bevorzugte Ausführungsform der erfindungsgemäßen Vorrichtung schematisch dargestellt. Zu den obenstehend beschriebenen Ausführungsformen identische Teile sind mit identischen Bezugszeichen bezeichnet. Zusätzlich befindet sich in dieser Ausführungsform der erfindungsgemäßen Vorrichtung stromabwärts vom Dialysator 18 außerhalb des Feldes 14 ein Adsorber 19.

Die dritte Leitungseinrichtung 24 gemäß der in Figur 5 dargestellten Ausführungsform weist ein erstes und ein zweites Ende auf, wobei das erste Ende mit dem Patienten P verbindbar ist und das zweite Ende mit dem Flüssigkeitszuführeingang 22 einer ungefilterten Seite eines Filters 20 verbunden ist. Der Plasmafilter 20 weist neben der ungefilterten Seite eine gefilterte Seite auf, wobei die ungefilterte Seite von der gefilterten Seite durch zumindest ein Filtermaterial getrennt ist. Die vierte Leitungseinrichtung 27 weist ein erstes und ein zweites Ende auf, wobei das erste Ende mit dem Flüssigkeitsabführausgang 26 einer ungefilterten Seite eines Filters 20 verbunden ist und das zweite Ende mit dem Patienten P verbindbar ist. Das Vollblut, welches durch den Flüssigkeitszuführeingang 22 der ungefilterten Seite eintritt, kann als Blutplasma teilweise durch den Flüssigkeitsabführausgang 28 der gefilterten Seite wieder austreten. Über die erste Leitungseinrichtung 10 wird dann das abgetrennte Blutplasma durch einen Dialysator 18 und einen Adsorber 19 geleitet. In dieser Ausführungsform wird nur der Dialysator 18 von einem Mittel zum Erzeugen eines Feldes 14 zumindest teilweise umgeben. Unter Einwirkung des Feldes werden die proteingebundenen Toxine zumindest teilweise freigesetzt und über die Membran des Dialysators 18 abgetrennt. Anschließend fließt das Blutplasma weiter über den Adsorber 19, der die im Feld freigesetzten Toxine aufnimmt. Anschließend fließt das Blutplasma in die zweite Leitungseinrichtung 12 und tritt am Flüssigkeitszuführeingang 29 der gefilterten Seite wieder in den Plasmafilter 20 ein und durch den Flüssigkeitsabführausgang 26 der ungefilterten Seite in die vierte Leitungseinrichtung aus. In der vierten Leitungseinrichtung 27 treffen somit das ungefilterte Vollblut und das bestrahlte und gereinigte Blutplasma wieder aufeinander. Diese Vorrichtung hat den Vorteil, dass neben dem Dialysator 18 zusätzlich ein Adsorber 19 die aus den Plasmaproteinen freigesetzten Toxine bindet. Die Anordnung des Adorbers im Plasmakreislauf (10, 12) hat den weiteren Vorteil, dass aus dem Adsorber freigesetztes Adsorbermaterial vom Plasmafilter zurückgehalten werden und nicht in den Vollblutkreislauf (24, 27) gelangen kann.

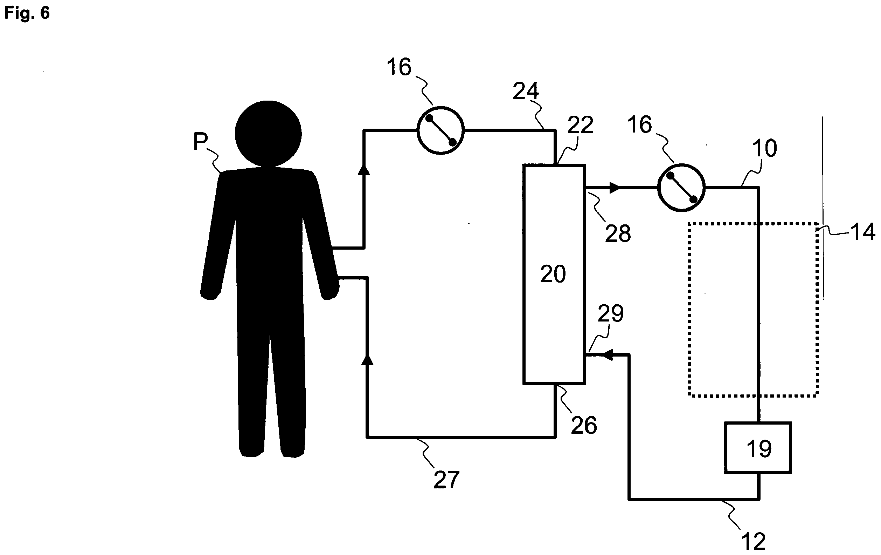

In Figur 6 ist eine weitere Ausführungsform der erfindungsgemäßen Vorrichtung schematisch dargestellt. Zu den obenstehend beschriebenen Ausführungsformen identische Teile sind mit identischen Bezugszeichen bezeichnet. In dieser Ausführungsform der erfindungsgemäßen Vorrichtung wirkt das elektrische Feld 14 auf die erste Leitungseinrichtung 10. Stromabwärts, außerhalb des Feldes 14, befindet sich ein Adsorber 19.

Die dritte Leitungseinrichtung 24 gemäß der in Figur 6 dargestellten Ausführungsform weist ein erstes und ein zweites Ende auf, wobei das erste Ende mit dem Patienten P verbindbar ist und das zweite Ende mit dem Flüssigkeitszuführeingang 22 einer ungefilterten Seite eines Filters 20 verbunden ist. Der Plasmafilter 20 weist neben der ungefilterten Seite eine gefilterte Seite auf, wobei die ungefilterte Seite von der gefilterten Seite durch zumindest ein Filtermaterial getrennt ist. Die vierte Leitungseinrichtung 27 weist ein erstes und ein zweites Ende auf, wobei das erste Ende mit dem Flüssigkeitsabführausgang 26 einer ungefilterten Seite eines Filters 20 verbunden ist und das zweite Ende mit dem Patienten P verbindbar ist. Das Vollblut, welches durch den Flüssigkeitszuführeingang 22 der ungefilterten Seite eintritt, kann als Blutplasma teilweise durch den Flüssigkeitsabführausgang 28 der gefilterten Seite wieder austreten. Über die erste Leitungseinrichtung 10 wird dann das abgetrennte Blutplasma durch und einen Adsorber 19 geleitet. In dieser Ausführungsform wird nur die erste Leitungseinrichtung 10 von einem Mittel zum Erzeugen eines Feldes 14 zumindest teilweise umgeben. Unter Einwirkung des Feldes werden die proteingebundenen Toxine zumindest teilweise freigesetzt. Anschließend fließt das Blutplasma weiter über den Adsorber 19, der die im Feld freigesetzten Toxine aufnimmt. Anschließend fließt das Blutplasma in die zweite Leitungseinrichtung 12 und tritt am Flüssigkeitszuführeingang 29 der gefilterten Seite wieder in den Plasmafilter 20 ein und durch den Flüssigkeitsabführausgang 26 der ungefilterten Seite in die vierte Leitungseinrichtung aus. In der vierten Leitungseinrichtung 27 treffen somit das ungefilterte Vollblut und das bestrahlte und gereinigte Blutplasma wieder aufeinander. Auch in dieser Ausführungsform hat die Anordnung des Adsorbers im Plasmakreislauf (10, 12) den Vorteil, dass aus dem Adsorber freigesetztes Adsorbermaterial vom Plasmafilter zurückgehalten wird und nicht in den Vollblutkreislauf (24, 27) gelangen kann.

Die nachfolgenden Versuchsergebnisse dienen als experimenteller Nachweis des Effektes eines elektrischen Feldes auf die Abtrennung proteingebundener Toxine während der Dialyse.

In Ausführungsbeispiel 1 wird der Effekt eines HF-Feldes im Frequenzbereich von 1 bis 20 MHz beschrieben. Ausführungsbeispiel 2 zeigt den Effekt des HF-Feldes im Frequenzbereich von 1 bis 170 MHz auf die Abtrennung von Phenylessigsäure. Unter dem Einfluss des HF-Feldes konnte die Abtrennrate für Phenylessigsäure um mindestens 45,3% gesteigert werden. Im Subband von 110 bis 120 MHz war der Effekt mit 54,6% besonders ausgeprägt. Im Ausführungsbeispiel 3 wird das Subband von 110 bis 120 MHz näher untersucht. Ausführungsbeispiel 4 zeigt den Einfluss eines H-Feldes in den Bereichen 1-6 MHz sowie 9-13 MHz. Ausführungsbeispiel 5 zeigt den Einfluss der Feldstärke auf die Abtrennung von Phenylessigsäure.

Die Temperatur wurde in allen Ausführungsbeispielen 1 bis 5 konstant gehalten, sodass die beobachteten Änderungen auf den Eigenschaften des elektrischen Feldes und nicht auf einer Erwärmung beruhen.

### Ausführungsbeispiel 1

In in-vitro-Versuchsreihen wurde der Einfluss hochfrequenter elektromagnetischer Felder auf den proteingebundenen Anteil der Urämietoxine untersucht.

Hierzu wurde ein Dialysemodul erstellt, indem konventionelle Hämofiltrations-Kapillaren mit Hilfe von Silikon als Schlaufen in einen Spritzenaufnahmestutzen eingegossen wurden. In das betreffende Modul wurde eine wässerige AlbuminLösung in Gegenwart der Urämie-Toxine Phenylessigsäure, p-Hydroxyhippursäure und Indoxylsulfat eingebracht. Mit Hilfe einer Spritzenpumpe wurde diese Lösung mit dem Dialysemodul für 10 min filtriert. Anschließend wurde durch Verwendung einer Hochfrequenz-Elektrode (HF-Elektrode) ein hochfrequentes elektromagnetisches Feld in der Lösung induziert. Das elektromagnetische Feld wird mittels einer Hochfrequenzspannungsquelle über einen Zeitraum von 10 Minuten von 1 bis 20 MHz in 1 MHz-Schritten inkrementiert. In den resultierenden Filtraten wurden die Konzentration der zuvor zum künstlichen Plasma gegebenen Urämie-Toxine Phenylessigsäure, p-Hydroxyhippursäure und Indoxylsulfat bestimmt. Durch Vergleich der Urämie-Toxin-Konzentrationen in den resultierenden Filtraten konnte der Effekt des HF-Feldes auf die Bindung zwischen Proteine und Urämie-Toxine evaluiert werden.

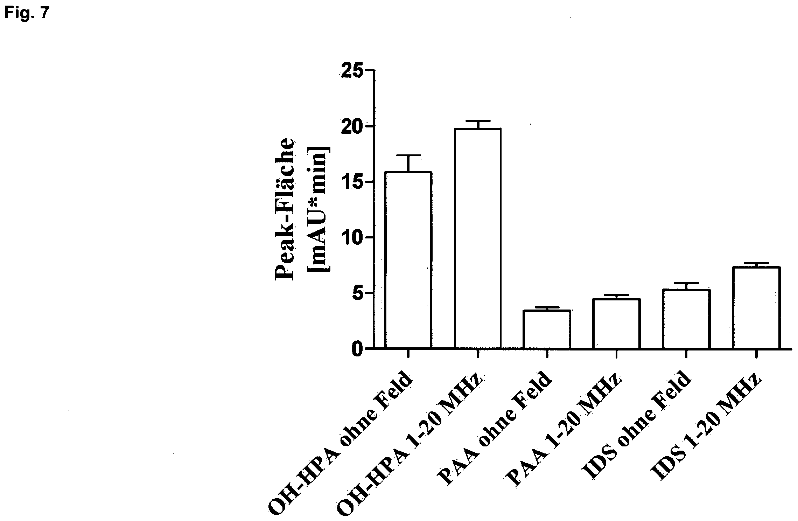

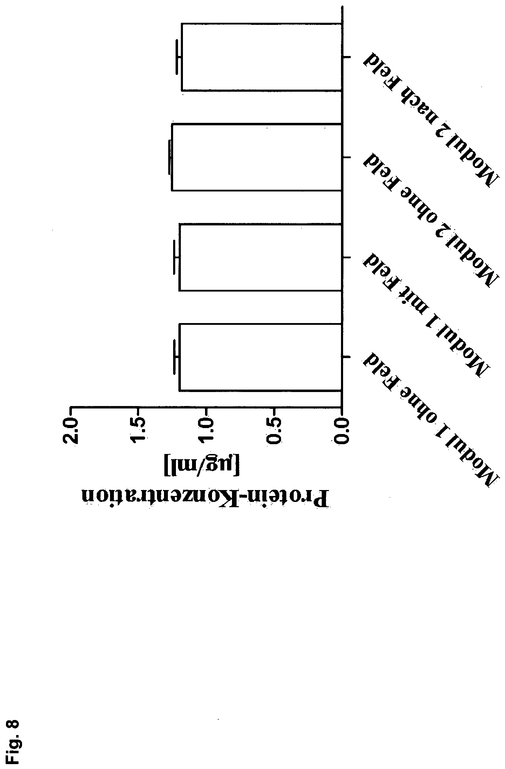

Die quantitative Bestimmung der Urämie-Toxin-Konzentration in den resultierenden Filtraten zeigte, dass hochfrequente elektromagnetische Felder die Filtrationsraten proteingebundener Urämie-Toxine signifikant erhöhen (Figur 7). Um zu überprüfen, ob hochfrequente elektromagnetische Felder die Dialysemembranen beschädigen, wurde die Proteinkonzentration im Filtrat mittels Proteinfärbung nach Bradford bestimmt. Die Ergebnisse zeigen, dass keine signifikanten Änderungen der Proteinkonzentration in Dialysemodulen ohne und unter Einfluss von hochfrequenten elektromagnetischen Feldern nachweisbar sind (Figur 8). Eine makroskopische Beschädigung der Membran ist aufgrund dieser Daten auszuschließen.

### Ausführungsbeispiel 2

Untersuchung der HF-Feldeinwirkung im Frequenz-Bereich 1 bis 170 MHz.

In das Dialysemodul aus Beispiel 1 wurde eine wässerige Lösung von bovinem Serumalbumin (BSA, 60 mg/ml) in Gegenwart des Urämie-Toxins Phenylessigsäure (1 mmol/l in 0.9 % NaCl-Lösung) eingebracht. Das HF-Feld wurde im Frequenz-Bereich 1-170 MHz in Subbändern von 10 MHz variiert und mit einem Kontrollversuch ohne HF-Feld verglichen.

Die quantitative Bestimmung der Phenylessigsäure erfolgte mittels HPLC.

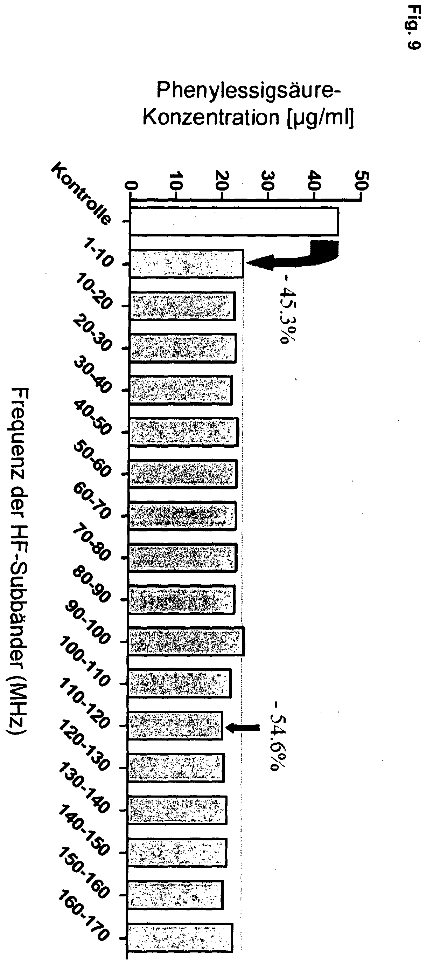

Die Ergebnisse der Versuche sind in Figur 9 zusammengefasst. Unter dem Einfluss des HF-Feldes konnte die Abtrennrate für Phenylessigsäure um mindestens 45,3% gesteigert werden. Im Subband von 110 bis 120 MHz war der Effekt mit 54,6% besonders ausgeprägt.

### Ausführungsbeispiel 3

Dieses Ausführungsbeispiel schließt an die Untersuchungen gemäß Ausführungsbeispiel 2 an, die gezeigt haben, dass der Effekt im Subband von 110 bis 120 MHz besonders ausgeprägt war.

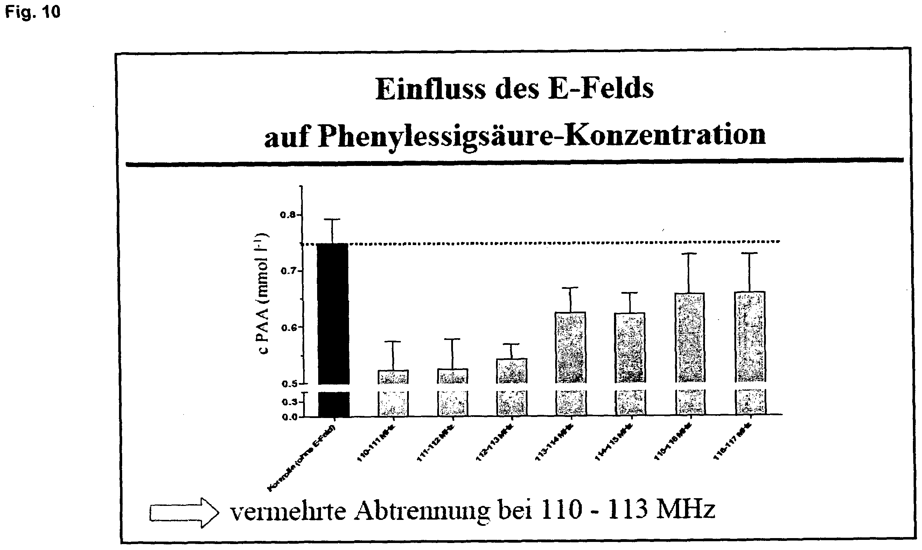

In den weiterführenden Untersuchungen gemäß Ausführungsbeispiel 3 konnte im Besonderen der Frequenzbereich um 110 bis 115 MHz als effektiver Frequenzbereich zur Freisetzung proteingebundener Urämietoxine identifiziert werden. Figur 10 zeigt den betreffenden Effekt auf die entsprechende Freisetzung und im Anschluss Abtrennung von Phenylessigsäure.

Nach bisherigem Stand eignen sich die im Folgenden in Tabelle 1 summarisch genannten Frequenzbereiche zur Abtrennung proteingebundener Urämietoxine.

**Tabelle 1: Geeignete Frequenzen im HF-Feld**

| **Frequenzen E-Feld** | **PAA** | **IDS** | **pCRS** |
|---|---|---|---|
| **80-120 MHz** | 110 | 110 | 110 |
| | 110-111 | 110-111 | 110-111 |
| | 111 | 111 | 111 |
| **120-170 MHz** | 140-141 | 140-141 | 140-141 |
| | 148-149 | | 151-152 |
| | 160-161 | | |

Bei den betreffenden Frequenzbereichen handelt es sich um die Bereiche, bei denen der maximale Abtrenneffekt bestimmt worden ist. In den nicht-genannten Frequenzbereichen wurde teilweise eine im Vergleich zu Kontrolle vermehrte Abtrennung bestimmt, die jedoch geringer war, als in den genannten Frequenzbereichen.

### Ausführungsbeispiel 4:

Weiterhin konnte auch im Bereich des H-Feld eine vermehrte Freisetzung und damit Abtrennung der proteingebundener Urämietoxine bestimmt werden.

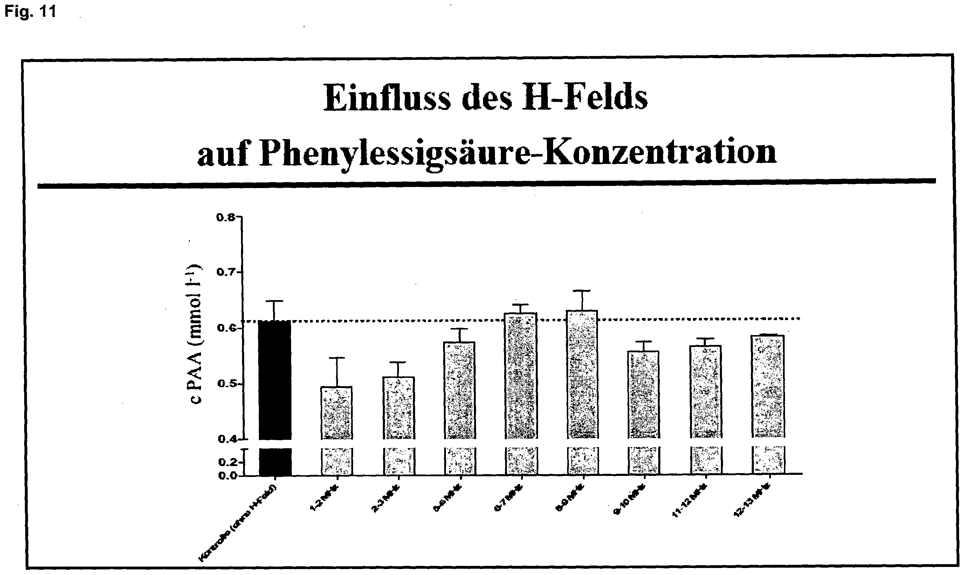

Figur 11 ist zu entnehmen, dass der H-Feld-Bereich von 1-6 MHz sowie der Bereich 9-13 MHz geeignet ist, proteingebundener Urämietoxine aus der Proteinbindung freizusetzen und in Folge dialytisch abzutrennen. Gezeigt ist in Figur 11 der Effekt auf Phenylessigsäure.

### Ausführungsbeispiel 5:

Neben der Frequenz des eingesetzten Feldes ist auch dessen Feldstärke für die resultierende Freisetzung und Abtrennung relevant. Mit zunehmender Feldstärke werden vermehrt die betreffenden Urämietoxine aus der Proteinbindung freigesetzt und anschließend abtrennt werden.

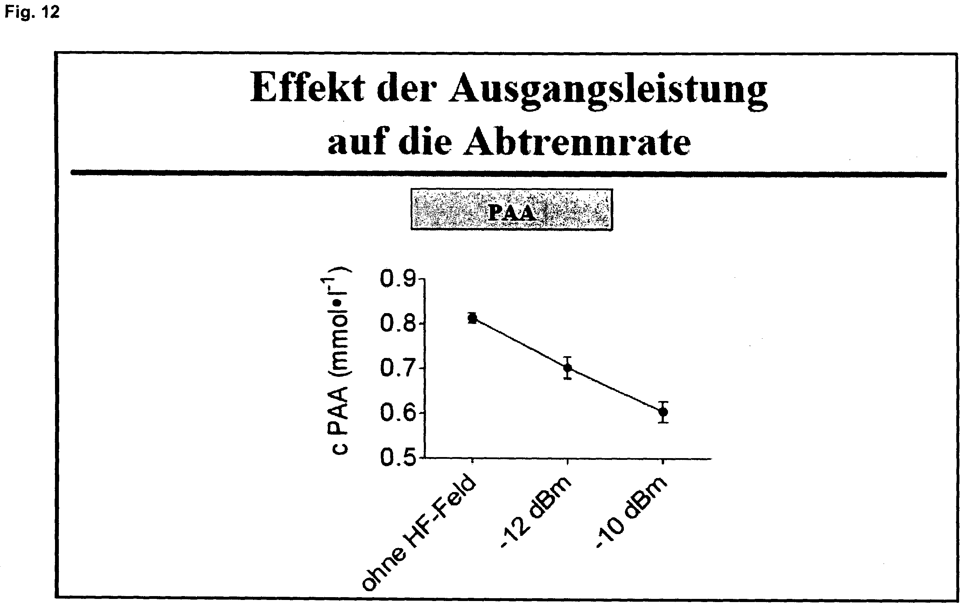

Figur 12 zeigt den Effekt einer ansteigenden Feldstärke auf den Gehalt proteingebundener Urämietoxine im Retentat am Beispiel der Phenylessigsäure.

## Patentansprüche

1. Vorrichtung zur extrakorporalen Entfernung proteingebundener Toxine aus Blutplasma, umfassend eine erste (10), zweite (12), dritte (24) und vierte (27) Leitungseinrichtung, einen zwischen der ersten (10) und der zweiten (12) Leitungseinrichtung angeordneten Dialysator oder Hämofilter (18) und/oder einen Adsorber (19), ein Mittel zur Erzeugung eines Feldes (14), das die erste Leitungseinrichtung (10) und/oder den Dialysator oder Hämofilter (18) und/oder den Adsorber (19) zumindest teilweise umgibt, eine steuerbare, in der ersten (10) und/oder zweiten (12) Leitungseinrichtung angeordnete Flüssigkeitsfördereinrichtung (16) und zumindest eine steuerbare in der dritten (24) und/oder vierten (27) Leitungseinrichtung angeordnete Körperflüssigkeitsfördereinrichtung (16), einen Plasma-Filter (20), wobei die Permeatseite des Plasma-Filters (20) mit der ersten (10) und der zweiten (12) Leitungseinrichtung verbunden ist und die ungefilterte Seite des Plasma-Filters (20) an ihrem Eingang (22) mit der dritten mit einem Patienten (P) verbindbaren Leitungseinrichtung (24) und an ihrem Ausgang (26) mit der vierten mit dem Patienten (P) verbindbaren Leitungseinrichtung (27) verbunden ist, worin mittels der Flüssigkeitsfördereinrichtungen (16) ein steuerbarer Durchfluss der Flüssigkeit durch die Leitungseinrichtungen (10, 12, 24, 27) und den Dialysator oder Hämofilter (18) und/oder den Adsorber (19) erzeugbar ist, **dadurch gekennzeichnet dass** das Mittel (14) ein hochfrequentes elektrisches Feld mit einer Frequenz von 100 kHz bis 2 GHz und/oder ein hochfrequentes elektromagnetisches Feld mit einer Frequenz von 100 kHz bis 2 GHz und/oder ein elektrisches Gleichfeld mit einer Feldstärke zwischen 10 V/m und 400 v/m erzeugt.

2. Vorrichtung nach Anspruch 1, wobei das Mittel (14) ein hochfrequentes elektromagnetisches Feld und ein elektrischen Gleichfeld erzeugt.

3. Vorrichtung nach Anspruch 1, wobei das Mittel (14) ein hochfrequentes elektrisches Feld und ein elektrischen Gleichfeld erzeugt.

4. Vorrichtung nach einem der vorhergehenden Ansprüche, wobei zwischen der ersten (10) und der zweiten (12) Leitungseinrichtung ein Dialysator (18) und ein Adsorber (19) angeordnet sind.

5. Vorrichtung nach einem der vorhergehenden Ansprüche, wobei zwischen der ersten (10) und der zweiten (12) Leitungseinrichtung ein Dialysator (18) angeordnet ist.

6. Vorrichtung nach einem der vorhergehenden Ansprüche, wobei zwischen der ersten (10) und der zweiten (12) Leitungseinrichtung ein Adsorber (19) angeordnet ist.

7. Vorrichtung nach einem der vorhergehenden Ansprüche, wobei das Mittel zur Erzeugung eines hochfrequenten elektrischen Feldes oder eines hochfrequenten elektromagnetischen Feldes (14) ausgewählt ist aus einer Hochfrequenzspule, einer Hochfrequenzelektrode und/oder einem Hochfrequenzkondensator.

8. Vorrichtung nach einem der vorhergehenden Ansprüche, wobei das hochfrequente elektrische Feld oder das hochfrequente elektromagnetische Feld eine Frequenz von 1 MHz bis 1 GHz aufweist.

9. Vorrichtung nach einem der vorhergehenden Ansprüche, wobei das elektrische Gleichfeld eine Feldstärke von 100 bis 250 V/m aufweist.

10. Vorrichtung nach einem der vorhergehenden Ansprüche zur Verwendung in der Behandlung des akuten oder chronischen Nierenversagens.

11. Vorrichtung nach einem der vorhergehenden Ansprüche zur Verwendung in der Behandlung des akuten oder chronischen Leberversagens.

## Claims

1. An apparatus for extracorporeal removal of protein-bound toxins from blood plasma comprising a first line device (10), a second line device (12), a third line device (24) and a fourth line device (27), a dialyzer or hemofilter (18) arranged between the first line device (10) and the second line device (12) and/or an adsorber (19), means for generating a field (14) at least partially surrounding the first line device (10) and/or the dialyzer or hemofilter (18) and/or the adsorber (19), a controllable fluid conveyance device (16) arranged in the first line device (10) and/or the second line device (12), and at least one controllable body fluid conveyance unit (16) arranged in the third line device (24) and/or the fourth line device (27), a plasma filter (20), wherein the permeate side of the plasma filter (20) is connected to the first line device (10) and the second line device (12) and the unfiltered side of the plasma filter (20) is connected at its inlet (22) to the third line device (24) which can be connected to a patient (P) and is connected at its outlet (26) to the fourth line device (27) which can be connected to the patient (P), wherein a controllable flow of fluid through the line devices (10, 12, 24, 27) and the dialyzer or hemofilter (18) and/or the adsorber (19) can be generated by means of the fluid conveyance devices (16), **characterized in that** the means (14) generates a high-frequency electric field having a frequency of 100 kHz to 2 GHz and/or a high-frequency electromagnetic field having a frequency of 100 kHz to 2 GHz and/or an electric DC field having a field strength between 10 V/m and 400 V/m.

2. The apparatus according to Claim 1, wherein the means (14) generates a high-frequency electromagnetic field and an electric DC field.

3. The apparatus according to Claim 1, wherein the means (14) generates a high-frequency electric field and an electric DC field.

4. The apparatus according to any one of the preceding claims, wherein a dialyzer (18) and an adsorber (19) are arranged between the first line device (10) and the second line device (12).

5. The apparatus according to any one of the preceding claims, wherein a dialyzer (18) is arranged between the first line device (10) and the second line device (12).

6. The apparatus according to any one of the preceding claims, wherein an adsorber (19) are arranged between the first line device (10) and the second line device (12).

7. The apparatus according to any one of the preceding claims, wherein the means for generating a high-frequency electric field or a high-frequency electromagnetic field (14) are selected from a high-frequency coil, a high-frequency electrode and/or a high-frequency capacitor.

8. The apparatus according to any one of the preceding claims, wherein the high-frequency electric field or the high-frequency electromagnetic field has a frequency of 100 kHz to 1 GHz.

9. The apparatus according to any one of the preceding claims, wherein the electric DC field has a field strength of 100 to 250 V/m.

10. The apparatus according to any one of the preceding claims for use in the treatment of acute or chronic renal failure.

11. The apparatus according to any one of the preceding claims for use in the treatment of acute or chronic hepatic failure.

## Revendications

1. Dispositif d'élimination extracorporelle des toxines liées aux protéines dans le plasma sanguin, comprenant un premier (10), deuxième (12), troisième (24) et quatrième (27) dispositif de conduit, un dialyseur ou hémofiltre (18) et/ou un adsorbant (19) disposé entre le premier (10) et le deuxième (12) dispositif de conduit, un moyen destiné à générer un champ (14), qui entoure au moins partiellement le premier dispositif de conduit (10) et/ou le dialyseur ou hémofiltre (18) et/ou l'adsorbant (19), un dispositif de transport de liquide (16) qui peut être commandé, disposé dans le premier (10) et/ou deuxième (12) dispositif de conduit et au moins un dispositif de transport de liquide corporel (16) qui peut être commandé, disposé dans le troisième (24) et/ou quatrième (27) dispositif de conduit, un filtre à plasma (20), le côté perméat du filtre à plasma (20) étant relié au premier (10) et au deuxième (12) dispositif de conduit et le côté non filtré du filtre à plasma (20) étant relié au niveau de son entrée (22) au troisième dispositif de conduit (24) pouvant être relié à un patient (P) et au niveau de sa sortie (26) au quatrième dispositif de conduit (27) pouvant être relié au patient (P), dans lequel, au moyen des dispositifs de transport de liquide (16), un écoulement régulable du liquide à travers les dispositifs de conduit (10, 12, 24, 27) et le dialyseur ou hémofiltre (18) et/ou l'adsorbant (19) peut être généré, **caractérisé en ce que** le moyen (14) génère un champ électrique à haute fréquence avec une fréquence de 100 kHz à 2 GHz et/ou un champ électromagnétique à haute fréquence avec une fréquence de 100 kHz à 2 GHz et/ou un champ de courant continu avec une intensité du champ comprise entre 10 V/M et 400 V/m.

2. Dispositif selon la revendication 1, dans lequel le moyen (14) génère un champ électromagnétique à haute fréquence et un champ de courant continu.

3. Dispositif selon la revendication 1, dans lequel le moyen (14) génère un champ électrique à haute fréquence et un champ de courant continu.

4. Dispositif selon l'une des revendications précédentes, dans lequel un dialyseur (18) et un adsorbant (19) sont disposés entre le premier (10) et le deuxième (12) dispositif de conduit.

5. Dispositif selon l'une des revendications précédentes, dans lequel un dialyseur (18) est disposé entre le premier (10) et le deuxième (12) dispositif de conduit.

6. Dispositif selon l'une des revendications précédentes, dans lequel un adsorbant (19) est disposé entre le premier (10) et le deuxième (12) dispositif de conduit.

7. Dispositif selon l'une des revendications précédentes, dans lequel le moyen destiné à générer un champ électrique à haute fréquence ou un champ électromagnétique à haute fréquence (14) est choisi parmi une bobine à haute fréquence, une électrode à haute fréquence et/ou un condensateur à haute fréquence.

8. Dispositif selon l'une des revendications précédentes, dans lequel le champ électrique à haute fréquence ou le champ électromagnétique à haute fréquence présente une fréquence de 1 MHz à 1 GHz.

9. Dispositif selon l'une des revendications précédentes, dans lequel le champ de courant continu présente une intensité du champ de 100 à 250 V/m.

10. Dispositif selon l'une des revendications précédentes destiné à être utilisé dans le traitement de l'insuffisance rénale aiguë ou chronique.

11. Dispositif selon l'une des revendications précédentes destiné à être utilisé dans le traitement de l'insuffisance hépato-cellulaire aiguë ou chronique.
